# EUROPEAN PATENT SPECIFICATION

(11) **EP 1 006 195 B1**
(45) Date of publication and mention of the grant of the patent: **19.03.2008**
(21) Application number: 98932182.3
(22) Date of filing: 09.07.1998
(51) Int. Cl.: C12N 15/83, C07K 14/08

(54) **INFECTIOUS VECTORS AND CLONES OF PLANTS DERIVED FROM THE TURNIP MOSAIC VIRUS (TuMV)**
INFEKTIÖSE VEKTOREN UND PFLANZENKLONE, DIE SICH VON TURNIP MOSAIKVIRUS HERLEITEN
CLONES ET VECTEURS INFECTIEUX DE PLANTES DERIVES DU VIRUS DE LA MOSAIQUE DU NAVET (TUMV)

(30) Priority: 09.07.1997 ES 9701522
(43) Date of publication of application: 07.06.2000
(73) Proprietor: INSTITUTO NACIONAL DE INVESTIGACION Y TECNOLOGIA, AGRARIA Y ALIMENTARIA, E-28003 Madrid (ES)
(72) Inventor: PONZ ASCASO, Fernando, E-28230 Madrid (ES); TORRES PASCUAL, Vicente, E-28029 Madrid (ES); SANCHEZ SANCHEZ, Florentina, E-28008 Madrid (ES); MARTINEZ HERRERA, David, E-28224 Pozuelo de Alarcon (ES)
(74) Representative: Gonzalez Vacas, Eleuterio
(86) International application number: PCT/ES1998/000200
(87) International publication number: WO 1999/002718

(56) References cited:
- EP-A- 0 693 555
- WO-A-95/12669
- KONG L.J. ET AL.: 'Turnip mosaic virus coat protein gene: Cloning and contruction of the plant rector' SCI. CHINA SER. B. CHEM. LIFE SCI. EARTH SCI., vol. 35, 1992, page 12
- NICOLAS O. ET AL.: 'The complete nucleotide sequence of turnip mosaic potyvirus RNA' JOURNAL OF GENERAL VIROLOGY, vol. 73, 1992, pages 2785 - 2793
- OSHIMA K. ET AL.: 'The complete nucleotide sequence of turnip mosaic virus RNA japanese strain' ARCHIVES OF VIROLOGY, vol. 141, 1996, pages 1991 - 1997
- CHIANG C.H. ET AL.: 'Infectivity assays of in vitro and in vivo transcripts of papaya ringspot potyvirus' BOTANICAL BULLETIN OF ACADEMIA SINICA, vol. 38, 1997, pages 153 - 163
- NICOLAS O. ET AL.: 'The use of PCR for cloning of large CDNA framents of turnip mosaic virus RNA and the capsid protein gene' JOURNAL OF VIROLOGICAL METHODS, vol. 32, 1991, pages 57 - 66

## Description

### SCOPE OF THE INVENTION

The invention refers to infective clones of the turnip mosaic virus (TuMV) UK1 isolate and to plant infectious viral vectors based on said infectious clones.

### BACKGROUND OF THE INVENTION

Infectious viral clones comprise a DNA molecule and some transcription regulating mechanisms, capable of synthesising a transcript which can infect plants of plant cells (protoplasts). Infectious clones, in themselves, constitute a very valuable tool in basic research in Virology and, besides, are the necessary starting element for the preparation of plant viral vectors.

Plant viruses show different types of genoma: single stranded DNA (for example, geminivirus), double stranded DNA (for example, pararetrovirus), positive polarity RNA, i.e., messenger direction (for example potyviurs), negative polarity RNA (for instance, rabdovirus), double polarity RNA (for instance tospovirus) or double stranded RNA (for instance reovirus). Recombinant DNA technology initially allowed the development of infectious clones of plant viruses with DNA genoma (geminivirus and pararetrovirus). Afterwards, thanks to the development of reverse transcriptases infectious clones have been obtained from numerous plant virus groups with positive polarity RNA genoma [Boyer, J.C. & Haenni, A.L. (1994). Infectious transcripts and cDNA clones of RNA viruses. Virology, 198, 415-426]. Infectious clones from the following potyviruses have been described:
- Sharka virus (PPV) [Maiss E., Timple U., Brisske Rode A., Lesemann D.E. & Casper R. (1992). Infectious in vivo transcripts of a plum pox potyvirus full length cDNA clone containing the cauliflower mosaic virus 35S RNA promoter. Journal of General Virology, 73, 709-712; Riechmann J.L., Lain S. & Garcia J.A. (1990). Infectious in vitro transcripts from a plum box potyvirus cDNA clone. Virology, 177, 710-126];
- Tobacco vein mottling virus (TVMV). [Domier L.L., Franklin K.M., Hunt A.G., Thoads R.E. & Shaw J.G. (1989). Infectious in vitro transcripts from cloned cDNA of a potyvirus, tobacco vein mottling virus. Proceedings of the National Academy of Sciences USA, 86, 3509-3513];
- Tobacco engraving virus (TEV) [Dolja V.V., McBride H.J.& Carrington J.C. (1992). Tagging of plant potyvirus replication and movement by insertion of 0-glucuronidase into the viral polyprotein. Proceedings of the National Academy of Sciences USA, 89, 10208-10212];
- Pea seedborne mosaic virus (PSbMV) [Johansen I.E., Dougherty W.G., Keller K.E., Wang D. & Hampton R.O. (1996). Multiple viral determinants affect seed transmission of pea seedborne mosaic virus in Pisum sativum. Journal of General Virology, 77, 3149-3154];
- Peanut stripe virus (PStV) [Flasinki S., Gunasinghe U.B., Gonzales R.A. & Cassidy B.G. (1996). The cDNA sequence and infectious transcripts of peanut stripe virus. Gene, 171, 299-300] ;
- Zucchini yellow mosaic virus (ZYMV) [Gal-On A. , Antignus Y., Rosner A. & Raccah B. (1991). Infectious in vitro RNA transcripts derived from cloned cDNA of the cucurbit potyvirus, zucchini yellow mosaic virus, Journal of General Virology, 72, 2639-2643; Galon A., Meiri E., Huet H., Hua W.J., Raccah B. & Gaba V. (1995). Particle bombardment drastically increases the infectivity of cloned DNA of zucchini yellow mosaic potyvirus, Journal of General Virology, 76, 3223-3227];
- Papaya ringspot potyvirus (PRSV) [Chiang C.H. & Yeh S.D. (1995). Construction of infectious in vitro transcripts of papaya ringspot potyvirus. 6th International Plant Virus Epidemiology Symposium, Ma'ale Hachamisha, Jerusalem, Israel, page 52]; and
- Potato A virus (PVA) [Puurand V., Valkonen J., Makinen K., Rabenstein F. & Saarma M. (1996). Infectious in vitro transcripts form cloned cDNA of the potato A potyvirus. Virus Research, 40, 135-140.

The genoma of most of the plant viruses comprises one or several positive polarity RNA molecules. The infectious clones of plant viruses with RNA genoma basically comprise a complementary DNA (cDNA) to the viral genomic RNA and a promoter sequence linked to said cDNA. This promoter sequence can either be that of a bacteriophage, which allows the obtaining of viral RNA by *in vitro* transcription by the corresponding RNA polymerase, or else that of A plant functional gene, which allows, after the introduction of the DNA into the plants, *in vivo* production of the corresponding effective viral RNA.

The obtaining of an full length infectious clone from an RNA template (the viral genoma of an RNA virus) implies, in the first place, the use of a reverse transcriptase in order to make a first cDNA chain, in the second place, the synthesis of double stranded cDNA by means of a DNA polymerase, and, in the third place, the linking of different cDNA restriction fragments, although this third stage is not always carried out because some full length infectious clones have been prepared by means of the polymerase (RT-PCR) reserve transcription - chain reaction. The obtaining of a full length infectious clone from an RNA template is a complicated procedure which shows many difficulties since (i) a reading error in the polymerases may take place during either of the first two stages, and (ii) errors may also occur in the linking of the restriction fragments either by the presence of exonucleases or else by the presence of restriction fragments, small and difficult to detect, for the enzyme being used, entailing both kinds of errors the obtaining of full length clones which are not infectious [Boyer, J.C. & Haenni, A.L. (1994). Infectious transcripts and cDNA clones of RNA viruses. Virology, 198, 415-426]. Another factor which also has an influence is the precision of the linking of the promoter to the cDNA. The infectivity is very much decreased, even with 5' extensions of 1 or 2 nucleotides, however, the 3' extensions do not have such a big influence in the biological activity (Boyer, J.C. & Haenni, A.L. (1994). Infectious transcripts and cDNA clones of RNA viruses. Virology, 198, 415-426]. On the other hand, the handling of full length viruses is very complex due to the unstableness problems which they show.

One of the main applications for plant infectious viral clones is their use in the construction of viral vectors. A plant infectious viral vector consists, in general, of a infectious viral clone modified in such a way that it..contains a heterologous nucleic acid sequence which is expressed after inoculating plants or plant cells with said vector.

Plant viral vectors are used to research molecular biology processes in plants and are useful as tools in order to study plant viral infections processes. Besides, plant viral vectors have been developed in order to substitute and/or insert genes and to show epitopes. Among the commercial applications of known plant viral vectors are the expression of the interpheron alphaD gene, the expression of alpha-tricosanthine (inhibitor of viral replication in the human immunodeffiency virus [HIV]), the expression of the peptide inhibiting the enzyme which converts angiotensine I, the presentation of epitopes of the flu virus, of HIV-1, of malaria epitopes, and the expression of antigenic peptides of the glosopeda virus and human rhinovirus 14 [Scholthof, H.B., Scholthof, K.B.G. & Jackson, A.O. (1996). Plant virus gene vectors for transient expression of foreign proteins in plants. Annual Review of Phytopathology, 34, 299-323]. On the other hand, plant infectious vectors represent an alternative to the obtaining of transgenic plants and show numerous advantages, among which are, for instance, high expression in a short period of time, the expression of genes which can make more difficult the regeneration or the growth of transgenic plants and the possibility of a transient expression.

Plant viruses have a limited and specific range of hosts. Most of the plant species are not infected by most viruses, i.e., each virus is capable of infecting a unique combination of plant species [Dawson, W.O. & Hilf, M.E. (1992). Host range determinants of plant viruses. Annual Review of Plant Physiology and Plant Molecular Biology, 43, 527-555]. This situation implies that, with the present knowledge level of the factors determining the host range, it is impossible to obtain universal plant viral vectors. Therefore, there exists the need of looking for new clones and plant infectious viral vectors which can be used to infect useful plants as models for the study of the expression of heterologous and epitope genes of pathogens. The invention provides some turnip mosaic virus infectious clones, as well as some viral vectors based on said infectious vectors, adequate for infecting plants of Arabidopsis thaliana (L.) Heynh.

The turnip mosaic virus, hereinafter TuMV (Turnip mosaic virus) belongs to the potyvirus family, which are flexuous viruses whose virons are made of only one positive polarity RNA molecule of approximately 10,000 nucleotides, and of approximately 2,000 copies of only one type of protein, the coat protein (CP). The RNA genoma has a 5' area and another 3' one which are not translated (of approximately 150 and 200 bases respectively) constituting the central part of only one open reading stage which, when is translated, gives rise to only one polyprotein which is processed by means of at least 3 proteases coded by the virus in 8-10 gene products . [Riechmann J.L., Lain S. & Garcia J.A. (1992). Highlights and prospects of potyvirus molecular biology. Journal of General Virology, 73, 1-16]. The full sequence of a Canadian isolate of TuMV has been published [Nicolas, O. &. Laliberté, J.F. (1992). The complete nucleotide sequence of turnip mosaic potyvirus RNA. Journal of General Virology, 73, 2785-2793] as well as that of a Japanese isolate [Oshima K., Tanaka M. & Sako N. (1996). The complete nucleotide sequence of turnip mosaic virus RNA Japanese strain. Archives of Virology, 141, 1991-1997].

### BRIEF DESCRIPTION OF THE FIGURES

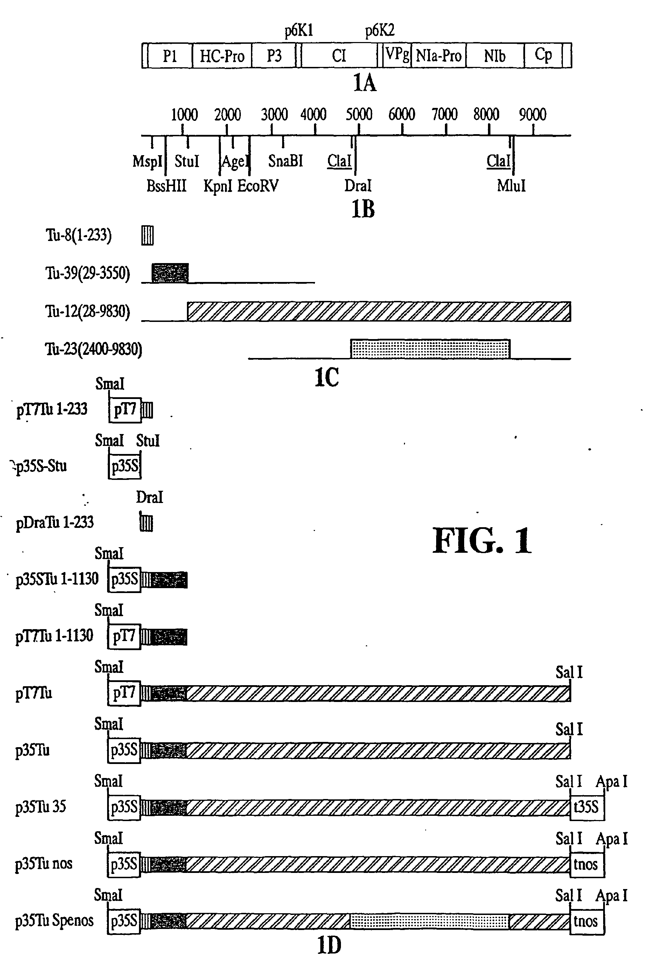

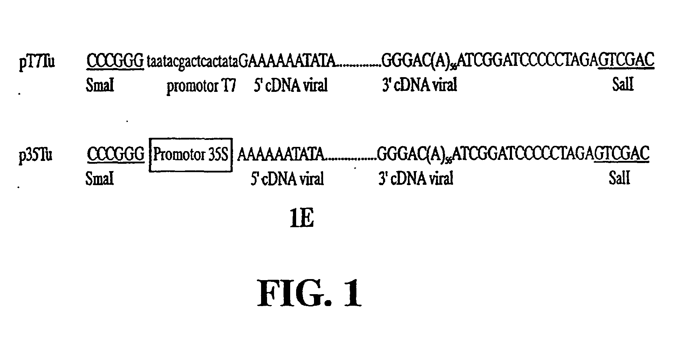

Figure 1 shows:
   - the genome organisation of TuMV, where the different genes are shown [P1: protein 1; HC-Pro: helper component protease; P3: protein 3; p6K1: 6 kilodalton peptide 1; CI: cylindrical inclusion : p6K2: : 6 kilodalton peptide 2; VPg: viral protein genome linked; Nia-Pro: small nuclear inclusion protein-protease; NIb: large nuclear inclusion protein; Cp: coat protein] (Figure 1A);
   - the scale (in nucleotides), the restriction map for unique cut points and the enzymes used in the construction of infectious clones [MspI, in bold, has not been mapped throughout the genoma, and ClaI, underlined, has two cutting sites] (Figure 1B);
   - the clones of cDNA or of RACE-PCR used in the construction of the infectious clones [the name of he clone is followed by positions 5' and 3' of the cDNA insert in parenthesis; the horizontal line indicates the area of the genome represented by the clone and the box with the weft represents the area of the clone used in the construction of infectious clones (Figure 1C);
   - infectious clones and intermediate clones in their construction (the sequences corresponding to the T7 and 35S promoter and to the nos and 35S terminators are indicated by boxes and their corresponding name (Figure ID); and
   - details at the nucleotide sequence level in the full length nucleotides, at the linking areas of cDNA to the promoters (5' end) or at the 3' end. The arrow points to the transcription initiation. The promoter T7 is shown at the nucleotide level. The 35S promoter is show in a box.

### DETAILED DESCRIPTION OF THE INVENTION

This invention relates to an infectious clone of the UK1 isolated of the turnip mosaic virus (TuMV), which comprises:
- a full copy of complementary DNA (cDNA) to the genomic RNA of TuMV UK1, in the shape of a double stranded DNA;
- a transcription promoter sequence operably linked to said cDNA, said promoter sequence being, selected from the group consisting of a cauliflower mosaic virus (CaMVI 35S promoter and a T7 RNA polymerase promoter; and
- a replicon.

In a particular embodiment, the TuMV is the UK1 isolate, and the full copy (cDNA) contains a poly-A tail at the 3' end.

The transcription promoter sequence or promoter, is a DNA sequence located at the 5'-terminal end and immediately before nucleotide (nt) 1 of the cDNA of the genomic RNA, onto which the RNA polymerase binds in order to initiate the transcription of messenger RNA (mRNA). Said transcription promoter sequence comprises the sequence of bacteriophage T7 and an additional G adjacent to the first nucleotide at the 5'end of the cDNA if said Cdna does not begin by G.

The infectious clon of the TuMV UK1 also comprises a transcription termination sequence or one signalling polyadenilation, for instance, the terminator of the nopaline sinthetase gene (nos) of the Ti plasmid of Agrobacterium tumefaciens or the polyadenilation signal of the 35S gene of CaMV. These termination sequences must be located at the 3' end of the poly(A) tail. Sometimes, there may be a variable number of nucleotides between the last nucleotide on the 3' end of the poly(A) tail and the first nucleotide of the transcription termination sequence or of the polyadenilation signal.

The replicon is a DNA molecule which has a replication origin and is, therefore, potentially capable of replicating inside of an adequate cell. An example of a replicon is a plasmid. The replicon is adequate for the maintenance and amplification of the infectious clone inside of an adequate host cell, such as a bacterial host, for instance, 'Escherichia coli (E. Coli). The replicon generally carries an antibiotic resistance gene which allows the selection and avoids the loss of the replicon as such or as a chimera after the insertion of the above mentioned elements. In a particular embodiment, the replicon is a plasmid chosen from pUC12 and pUC13.

Example 1 describes the construction of three infectious clones of the UK1 isolate of the TuMV virus. The three of them have the same full length sequence, cDNA, of the genoma of TuMV UK1. One of the clones is adequate for the inoculation with RNA, since the cDNA is under the control of the promoter of the bacteriophage T7, while the other two clones are adequate for the DNA inoculation, they have the same promoter, promoter 35S of CaMV, but one of them has the CaMV 35S polyadenilation signal and the other one the nos terminator gene.

The procedure for the obtaining of said infectious clones entails the construction of cDNA, full length, of the genomic RNA of the potyvirus and the assemblage of transcription regulating elements (promoters and, if so be the case, terminators).

In a particular embodiment (cf. Examples 1.1. and 1.2), the full length cDNA of the genomic RNA of TuMV UK1, has been constructed by means of the linking of three different fragments:
a) a fragment of approximately 8.7 kilobases (kb) coming from a cDNA from TuMV UK1 of 9.8 kb obtained by standard synthesis of cDNA used as (3') reverse primer, a d(T)36 oligo-identified as SEQ. ID. NO.; 4 (cf. the LIST OF SEQUENCES);
b) a fragment of 900 nt, internal to a clone of cDNA of 3.55 kb of TuMV UK1 obtained by the standard synthesis of cDNA using as (3') reverse primer an oligonucleotide complementary to nt 4769-4747 of the Canadian TuMV isolate, identified as SEQ. ID. NO.: 2; and
c) the first 200 nt, generated by means of RT-PCR, with a specific (3') reverse primer complementary to the 233-212 positions of the TuMV UK1 genoma, called Tu 233-212 and identified as SEQ. ID. NO.: 3, and a (5') direct primer from a commercial kit (5' ampliFINDER RACE kit, Clontech).

These fragments have been linked, in the manner indicated in the examples, in order to get the full length cDNA of the viral genomic RNA.

In the sense used in this description, the expression. "Turnip mosaic virus (TuMV) UK1" refers to a TuMV with a serotype that it groups isolates which reacts positively with EMA 58, EMA 70 and EMA 67 monoclonals antibodies, and does not reacts with the EMA 84 and EMA 115 monoclonals antibodies, (Jenner, C. E., Keane, G. J., Jones, J. E. & Walsh, J. A. (1999). "Serotypic variation in Turnip Mosaic Virus". Plant Pathology 48, 101-108).

TuMV UK1 also refers to a TuMV with a pathologic profile that belongs to group 1, when are carried out analysis of the reactions of different rape lines and swede lines, (Walsh, J. A. 1989. "Genetic control of immunity to turnip mosaic virus in winter oilseed rape (Brassica napus spp. oleifera) and the'effect of foreign isolates of the virus." Annals of Applied Biology 115: 89-99). Depending on to capacity to infect certain rape (S6 o R4) lines o swede lines (165 o S1), TuMV UK1 also refers to a TuMV with a pathologic profile that belongs to patotype 1, (Jenner, C. E. and J. A. Walsh (1996). "Pathotypic variation in turnip mosaic virus with special reference to European isolates." Plant Pathology 45(5): 848-856).

In the sense used in this description, the expression "standard cDNA synthesis" refers to synthesis by means of reverse transcriptase and the second chain by means of the action of RNasaH and DNA polymerase.

Some of the elements or sequences used for the construction of the infectious clones provided by this invention are products known and available in the market, such as the replicon (plasmids pUC13 and pUC12), the T7 promoter sequence, the CaMV 35S promoter sequences and the polyadenilation signal (from a commercial plasmid, by PCR amplification by specially designed primers) and the sequence of the nos gene terminator (by means of the amplification by PCR of a commercial plasmid with specially designed primers).

TuMV UK1 infectious clones provided by this invention are adequate for basic research in virology and for the construction of plant viral vectors.

An additional subject matter of this invention relates to a recombinant viral vector derived from a TuMV UK1 infectious clone provided by this invention. Said viral vector comprises a TuMV UK1 infectious clone modified in order to contain a gene or a heterologous gene fragment in said infectious clone under conditions which allow the expression of said gene or gene fragment after the infection of a plant or a protoplast by said recombinant viral vector.

Heterologous genes or fragments which the viral vectors provided by this invention may contain, may comprise any gene or fragment which codes for a protein, a peptide, an epitope or any gene product of interest. Said genes or heterologous fragments can be introduced into the infectious clone by genetic engineering techniques. It is essential in the construction of viral vectors, that the introduction of the heterologous gene or fragment does not interfere with any of the basic viral functions. A very interesting application of plant infectious viral vectors is the expression in plants of animal viral epitopes in order to produce edible vaccines against said animal viruses.

The invention also provides a method to infect plants which entails the putting together of one of the TuMV UK1 infectious clones provided by this invention or a viral infectious vector derived from said clone with a plant susceptible of being infected by TuMV UK1. In a particular embodiment, the plant belongs to the Arabidopsis thaliana (L.) Heynh species.

The following Examples serve the purpose of illustrating the invention and should not be considered as limiting of the scope of the same.

### EXAMPLE 1

### Infectious clone construction

### 1.1.Obtaining of cDNA from TuMV UK1

The TuMV isolate used is isolate TuMV UK1, donated by Dr. J. Walsh (HRI, Wellesbourne, United Kingdom). The TuMV UK1 isolate was purified from Indian mustard plants (Brassica juncea) inoculated with said virus three weeks before harvesting. The RNA extracted from the virus was used as the template for the synthesis of cDNA. Said synthesis was carried out, basically, according to the protocol described in the Amersham kit (cDNA synthesis module kit), with some modifications. Reverse transcriptase superscript RNaseH⁻ (Gibco-BRL) was used for the synthesis of the first chain, using as primers those identified as SEQ. ID. NO.: 1 [cf. the SEQUENCE LIST] which corresponds to a d(T)14 oligo and as SEQ. ID. NO: 2, an oligonucleotide complementary to the nucleotide sequence 4769-4747 of Canadian TuMV isolate. The cDNA fragments where cloned into pUC13 [Messing, J. (1983). New M13 vectors for cloning. Methods in Enzymology, 100 B, 20-78] digested with Smal and treated with phosphatases (shrimp alkaline phosphatase, USB]. Clones were analysed by restriction with different endonucleases and by determination of the sequence at the ends. The position of the nucleotides corresponding to the viral genoma were assigned to the ends of the inserts, by similarity to the sequence of the complete genoma of another TuMV isolate [Nicolas, O. & Laliberté, J.F. (1992). The complete nucleotide sequence of turnip mosaic potyvirus RNA. Journal of General Virology, 73, 2785-2793]. Two clones were selected (Tu-23 2400-9830 and Tu-39 29-3550) which contain overlapping-inserts which represent the almost full length cDNA of TuMV UK1, except for the 28 first nucleotides of the 5' end.

The cDNA corresponding to the 233 nt of the 5' end was synthesised by PCR using an oligonucleotide complementary to positions 233-212 of the UK1 genoma (Tu 233-212) as the primer; identified as SEQ. ID. NO.: 3 and the (5') direct primer ampliFINDER RACE (Clontech) under the following conditions:
- number of cycles:: 35
- temperature/time:: 45 seconds (s) at 94° C
45 s at 60° C, and
2 minutes at 72° C

The PCR fragments were cloned, after blunting the ends with T₄ DNA polymerase and introducing a phosphate at 5' by means of the T₄ polynucleotide kinase. The sequence of the 5' viral end was determined from said clones and also directly from the viral RNA using the oligonucleotide identified as SEQ. ID. NO.: 3 and the RT RNA sequencing kit (USB) as primers. The sequence of the ten clones was completely coincident except that they showed length heterogeneity at the 5' end (4, 5 or 6.As). A clone was selected, named Tu-8, which had 6 As at the 5' end.

### 1.2 cDNA synthesised with a d(T) 36 oligo primer

The analysis of the preceding cDNA clones indicated the presence of some short poly (A) tails (of approximately 14 nt) at the 3' end. A cDNA was synthesised using for the primer in the synthesis of the first chain the primer known as SEQ. ID. NO.: 4, a d(T)36 oligo (New England Biolabs), and said cDNA was cloned into a modified pUC13 vector which contained the following multiple cloning site:

HindIII-PstI-SalI-KpnI-EcoRV-BamHI-SmaI-SacI-EcoRI, digested with EcoRV and treated with phosphatase. The analysis of the clones thus obtained indicated the presence of a variable length (9-56 nt) poly(A) tail. Among these clones was a clone named Tu-12, with an insert size of about 9.8 kilobases (kb) approximately and a poly(A) tail of 56 residues, and said clone covered the genome of TuMV UK1 from nucleotide 28.

### 1.3. Restriction map of cDNA

In order to determine the restriction map of the full length cDNA (by overlapping of the restriction maps of the Tu-8, Tu-39 and Tu-23 clones) of TuMV UK1, the following enzymes were used: AflII, AgeI, BamHI, BssHII, ClaI, DraI, Eco47III, EcoRI, EcoRV, HindIII, HpaI, KpnI, MluI, NruI, NsiI, PstI, SacI, SnaBI, SpeI, StuI, XbaI, XhoI. No cutting sites have been detected for the following enzymes: AccIII, ApaI, EagI, NcoI, GgoMI, NotI, SalI, SmaI, SacII.

Table 1 shows the number of cutting sites and the approximate location in the cDNA derived from the TuMV genoma of the cutting sites for the restriction enzymes assayed.

**Table 1**

| **Restriction map** | | |
|---|---|---|
| Enzyme | No. | Site |
| AflII | 3 | 2500, 3700, 5700 |
| Agel | 1 | 2100 |
| BamHI | 2 | 2600, 6100 |
| BssHI | 1 | 600 |
| ClaI | 2 | 4700, 8400 |
| DraI | 1 | 4900 |
| Eco 47III | 2 | 3100, 8600 |
| EcoRI | 5 | 1100, 1700, 5300, 8300, 9300 |
| EcoRV | 1 | 2550 |
| HindIII . | 3 | 2600, 3800, 5800 |
| HpaI | 2 | 1000, 8500 |
| KpnI | 1 | 1900 |
| MluI | 1 | 8600 |
| NruI | 2 | 1300, 5300 |
| NsiI | 3 | 2100, 5100, 9600 |
| PstI | 2 | 5500, 6100 |
| SacI | 2 | 5700, 7900 |
| SnaBI | 1 | 3300 |
| SpeI | 5-6* | 2440, 2900, 3300, 3800, 6900, 7100* |
| StuI | 1 | 1130 |
| XbaI | 4 | 2800, 4900, 5300, 8500 |
| XhoI | 4 | 1800, 3700, 5600, 8400 |

| | | |
|---|---|---|
| *: the cutting site Spel at the 7100 position is only present in certain cDNA clones, and absent (or minoritarious) in the viral population (in the viral genoma). | | |

### 1.4 Fusion of the 35S Promoter to the viral cDNA

The 35S promoter [432 base pairs (pb)] of the CaMV was amplified by PCR using the pRT101 plasmid as the template [Topfer, R., Matzeit, V., Gronenborn, B., Schell, J., and Steinbiss, H.H. (1987). A set of plant expression vectors for transcriptional and translational fusions. Nucleic Acids Research, 15 (14): 5890], as the (5') direct primer, the one identified as SEQ. ID. NO: 5 and as the (3') reverse primer the one identified as SEQ. ID. NO.: 6, under the following conditions: heating to 94° C for 5 minutes and then 30 30 s. cycles at 94° C, 1 minute at 62° C and 5 minutes at 72° C.

The amplified fragment was treated with T4 DNA polymerase, was phosphorilated with T4 polynucleotide kinase, was digested with BamHI, was subjected to electrophoresis in polyacrilamide gel (PAA) with 5% TBE (Tris 0.089 M, Borate 0.089 M, EDTA 0.002 M), was purified from the gel [Sambrook J., Fritsch E.F. & Maniatis T. (1989). Molecular cloning: A laboratory manual. Cold Spring Harbor Laboratory Press, New York; Smith H.O. (1980). Recovery of DNA from gels. Methods in Enzymology 65, 371-380], and was cloned into the pUC13 vector digested with SmaI-BamHI. The primers were designed in order for the fragment to have a SmaI site at the 5' end and a StuI site right before the transcription initiation site, following the general strategy normally used to fuse the CaMV 35S promoter (p35S) to the first viral nucleotide [Ding, S.W., Rathjen, J.P., Li, W.X., Swanson, R. Healy, H. & Symons, R.H. (1995): Efficient infection in a new plasmid vector. Journal of General Virology, 76, 459-464.; Yamaya, J., Yoshioka, M. Meshi, T. Okada, Y. & Ohno, T. (1988). Expression of tobacco mosaic virus RNA in transgenic plants. Molecular & General Genetics, 211, 520-525]. The new plasmid was called p35S.Stu.

The cDNA from TuMV UK1 present in the Tu-8 plasmid was amplified by PCR using the primers identified as (i) SEQ. ID. NO.: 7, a 25 base oligonucleotide (Dra-Tul-22) of which, the first three nucleotides at the 5' end are three additional nucleotides meant to create a DraI restriction site, and the 22 nucleotides left correspond to the first 22 nucleotides at the 5' end of the TuMV UK1 cDNA and (ii) SEQ. ID. NO.: 3. The conditions were the following: 94° C for 5 minutes; 5 30 s. cycles at 94° C, 30 s at 42° C and 3.0 s at 72° C, and 35 30 s cycles at 94° C and 45 s at 60° C. The amplified fragment (236-pb) was treated with the T4 DNA polymerase, was phosphorilated with T4 polynucleotide kinase, was purified from PAA and was cloned into pUC13 digested with SmaI and dephophorilated with alkaline phosphatase. One clone, the one named pDra Tul-233 was selected after checking that it had the adequate insert.

The DraI-MspI (191 pb) fragment of the pDra-Tu1-233 clone and the MspI-MspI (939 pb) fragment of the Tu-39 clone were linked to p35S-StuI, digested with StuI and treated with phosphatase. From this cloning, the p35s Tu 1-1130 clone was selected, which contains the cDNA from TuMV UK1 from nucleotide 1 to 1130 under the control of the promoter 35S. Low yield was detected in the purification of this plasmid; the cloning of the SacI-HindIII fragment from this plasmid at pUC12 gave as result the plasmid p35S Tu 1-1130 (+) (+ indicates that the TuMV gene' and the beta-gal insert have the same transcription direction), which showed less yield problems.

### 1.5 Fusion of the T7 promoter to the viral cDNA

The T7 promoter was fused to the first nucleotide of the cDNA of TuMV UK1 by means of the PCR amplification of the fragment present at the Tu-8 clone using the primers identified as (i) SEQ. ID. NO.: 3 and (ii) SEQ. ID. NO.: 8, named T7-Tul-22, a 43 base oligonucleotide of which the first three at the 5' end are used to reconstruct a SmaI site, the 17 following ones for the sequence corresponding to the T7 promoter and the additional G to include a good transcription initiation residue, and the 22 nucleotides left correspond to the first 22 nucleotides of the 5' sequence of the cDNA of UK1. The conditions were the following: 94° C for 5 minutes; 5 30 s. cycles at 94° C, 30 s at 42° C and 30 s at 72° C, and 35 30 s cycles at 94° C and 45 s at 60° C. The amplified fragment (254 pb) was treated with the T4 DNA polymerase, was purified from PAA, was phosphorilated with T4 polynucleotide kinase and was cloned into pUC13 digested with SmaI and treated with phosphatase. One clone, the one named pT7-Tul-233 was selected, containing the adequate sequence. From the pT7-Tul-233 clone the fragment pT7 Tul-191 (SmaI-MspI ends) was extracted, from the clone Tu-39 the fragment Tu 191-1130 (MspI-StuI ends), and these fragments were linked to the vector fragment (which does not contain the p35S). From this ligate the plasmid pT7 Tu 1-1130 plasmid was selected. Low yield was also detected in the purification of this plasmid; the cloning of the SacI-HindIII fragment of this plasmid into pUC12 gave as a result the plasmid pT7 Tul-1130 (+) (+ indicates that the TuMV insert and the beta-gal gene have the same transcription direction), which showed less yield problems.

### 1.6 Fusion of the terminators

The transcription terminators of the (tnos) gene and the polyadenilation signal of 35S (t35S) were obtained by PCR from commercial plasmid templates (see below). In both cases the primers were designed in order that, after the cloning at HindIII, they had a SalI cutting site at the 5' end and ApaI and XhoI sites at the 3' end.

In the case of the 35S polyadenilation signal of the CaMV, the template used was the pRT101 plasmid [Töpfer, R. Matzeit, V. Gronenborn, B., Schell, J., & Steinbiss (1987). A set of plant expression vectors for transcriptional and translational fusions. Nucleic Acids Research, 15 5890) and the primers used were those identified as (i) SEQ. ID. NO.: 9, a direct 22 base primer and (ii) SEQ. ID. NO.: 10, a reverse 30 base primer. The amplified fragment had 205 pb.

For the nos gene terminator (tnos), the template was the plasmid pBI2221 (Clontech) and the primers those identified as (i) SEQ. ID. NO.: 11 a 28 base oligonucleotide (direct primer) and (ii) SEQ. ID. NO.: 12, a 29 base oligonucleotide (reverse primer). The amplified fragment had 265 pb.

The amplification reaction in both cases was carried out under the following conditions: 3 minutes at 95° C; 35 30 s cycles at 95° C, 30 s at 52 °C and 1 minute at 72° C; and finally 5 minutes at 72° C.

The amplified fragments were treated with T4 DNA polymerase, were phosphorilated with T4 polynucleotide kinase, were purified from PAA, and were cloned into pUC13 digested with Hindi and treated with phosphatase. The clones pt35S and ptnos were identified which contained the correct fragments.

### 1.7 Assemblage of the full length clone under the control of p35S

The plasmid p35S Tul-1130 (+) [Example 1.4] was digested with XbaI, the 5' sticky ends were blunted by treating with the Klenow fragment of the DNA polymerase of Escherichia coli, was then cleaved with StuI and was dephosphorilated with alkaline phosphatase. This vector was ligated to the StuI-SmaI fragment of the Tu-12 clone (8.7 kp, nt 1130-9830 of cDNA from TuMV UK1). From this ligation, the clone named p35 Tu was selected, which contains the full length cDNA from TuMV UK1 under the control of promoter 35S.

### 1.8 Assemblage of the full length clone under the control of pT7

The SmaI-BssHII fragment of the p35 Tu plasmid (containing p35S and cDNA from TuMV UK1 nt 1-600) was substituted by a SmaI-BssHII fragment of the pT7 Tu 1-1130 plasmid. From the resulting construction the plasmid called pT7 Tu was selected which contains the full length cDNA from the genoma of TuMV UK1 under the control of the T7 bacteriophage promoter.

### 1.9 Introduction of the p35 Tu terminators

The fragments which contained the terminator of the gene nos (tnos) or the polyadenilation signal of 35S (t35S) were cleaved from the ptnos or pt35S plasmids [Example 1.6] by means of the double SalI-XhoI digestion, were purified from PAA, and were ligated to the p35 Tu plasmid digested with Sail and treated with phosphatase. From these ligations the clones p35 Tu 35 and p35 Tu nos were selected. These clones contained the same full length sequence, cDNA, of the TuMV UK1 genoma, as well as the same promoter, 35S, whilst one of them, p35 Tu 35, contained the t35S one and the other contained the tnos one.

From the p35 Tu nos clone, the p35 Tu Spe nos clone was obtained by substituting a ClaI fragment of p35 Tu nos by the Tu-23 equivalent fragment.

### 1.10 Stability test of the clones

This Example was carried out in order to check the stability of the infectious previously prepared clones of TuMV [see Examples 1.8 and 1.9] since, as it is known, the infectious full length clones of potyvirus are difficult to handle.

In the case of the full length clones previously prepared, both the one which is under the control of the T7 promoter and those under the control of the 35S promoter of CaMV, behave as unstable plasmids in Escherichia coli DH5 alpha isolate when they are grown at the optimal growing temperature for E. coli (37° C). This unstableness implies the loss, in a high percentage of the bacterial population, of the plasmid when the carrier isolate of said plasmid is cultured in a rich liquid medium (in the presence of selective pressure for the plasmid, i.e., ampicillin). In the solid media cultures in the presence of selective pressure (ampicillin), the unstableness implies the appearance of bacterial colonies with a different morphology (irregular edges, yellowish colour) from the one initially shown (smooth edges, translucent); these colonies different in morphology do not contain plasmids or else it is not of the adequate size. The described unstableness in the liquid and solid media disappears when the cultures are carried out at a temperature of 28-32° C.

### EXAMPLE 2

### Plant infection test

This Example was carried out in order to check the infection of plants using either an *in vitro* or *in vivo* transcript of TuMV UK1 through the usual techniques for plant inoculation.

For the performance of this test Arabidopsis thaliana (L.) Heynh RLD ecotype plants were used. The following were used as inoculum:
- purified DNA solutions derived from the p35 Tu 35 and p35 Tu nos plasmids [Example 1.9], with a concentration of 0.6 to 2 mg/ml, obtained after the alkaline lysis of a 250 ml culture in LB media (Sambrook J., Fritsch E.F. & Maniatis T. (1989). Molecular cloning: A laboratory manual. Cold Spring Harbor Laboratory Press, New York) grown at 30° C, treated with RNase, phenol and resuspended in TE buffer (10 mM Tris-HC1, 1 mM, pH 8.0 EDTA); and
- an *in vitro* transcript RNA solution using the plasmid T7 made linear by SalI as template and a commercial *in vitro* transcription kit (T7 cap scribe, from Boehringer and mMessage mMachine from Ambion). The transcription was carried out in a 360 □1 volume, with 20 □g of template plasmid (pT7 Tu) purified by CsCl and digested with SalI.

As infection controls TE pH 8.0 buffer and purified virus (TuMV) diluted to a concentration of 100 □g/ml in 50 mM pH 7.5 phosphate buffer were used.

In order to carry out this test, the seeds were sterilised, and were sown onto GM media plates (Valvekens D., van Montagu M. & van Lijsebettens M. (1988). Agrobacterium tumefaciens- mediated transformation of Arabidposis thaliana root explants by using kanamycin selection. Proceedings of the National Academy of Sciences USA, 85, 5536-5540) and were vernalised for 24 hours at 4° C. Then, they were moved to an *in vitro* culture chamber (16 hour photoperiod, 100 □E m⁻² s⁻¹ light intensity for 15 days). Once this period of time had elapsed they were transplanted onto soil (universal soil [Floragard] and vermicultite 3:1) and were kept in the culture chamber in the same light conditions and at a temperature of.22 **°** C.

Then, the plants were sprayed with an abrasive, such as silicon carbide. Two leaves of each, plant were inoculated with the previously mentioned inoculums (purified DNA derived from p35 Tu 35 and p35 Tu nos plasmids and *in vitro* RNA transcript solutions). In every case the DNA or RNA solutions were soaked onto a cotton wad and were rubbed onto the leaves to be inoculated.

The results of the different experiments (at least 2 different experiment for each type of inoculum) are indicated in Table 2.

**Table 2**

| Inoculum | Infected plants/inoculated plants | Infection percentage |
|---|---|---|
| Virus | 16/16 | 100 |
| Buffer | 0/16 | 0 |
| RNA transcript + "cap". like structure linear pT7 Tu template | 3/56 | 3 |
| p35 Tu 35 DNA | 6/36 | 16 |
| P35 Tu nos DNA | 13/35 | 37 |

Alternatively, this test could have been carried out by inoculating plant protoplasts with transcript RNA *in vitro* or with DNA [Leiser, R.M., Ziegler-Graff, V., Reutenauer, A., Herrbach, E., Lemaine, O., Guilley, H., Richards, K. & Jonard, G. (1992). Agroinfection as an alternative to insects for infecting plants with beet western yellows luteovirus. Proceedings of the National Academy of Sciences of the United States of America, 89, .9136-9140]

### MICRO-ORGANISM DEPOSITORY

Escherichia coli K12 DH5 alpha strands carriers of the pT7 Tu and p35 Tu nos plasmids have been deposited at the Colección Espanola de Cultivos Tipo (CECT), Burjasot, Valencia (España), on 19^{th} December 1996, being assigned access numbers CECT 4822 and CECT 4823 respectively.

### SEQUENCE LISTING

(1) GENERAL INFORMATION:
   (i) APPLICANT:
      (A) NAME: INSTITUTO NACIONAL DE INVESTIGACI6N Y TECNOLOGIA AGRARIA Y ALIMENTARIA
      (B) STREET: José Abascal, 56
      (C) CITY: Madrid
      (D) COUNTRY: Spain
      (E) ZIP CODE: 28003
      (F) TELEPHONE: (91) 347 39 33
      (G) TELEFAX: (91) 347 39 31
   (ii) TITLE OF THE INVENTION:
      PLANT INFECTIOUS CLONES AND VECTORS DERIVED FROM THE TURNIP MOSAIC VIRUS (TuMV)
   (iii) NUMBER OF SEQUENCES: 12
   (iv) COMPUTER READABLE FORM:
      (A) SUPPORT TYPE: Diskette
      (B) COMPUTER: IBM PC Compatible
      (C) OPERATING SYSTEM: PC-DOS/MS-DPS
      (D) SOFTWATE: Patent In Release #1,0, Version # 1.30
   (vi) PRIORITY DETAILS:
      (A)APPLICATION NUMBER: ES P9701522
      (B)APPLICATION DATE: 09-JUL-1997
(2) INFORMATION ON SEQ ID NO.: 1:
   (i) CHARACTERISTICS OF THE SEQUENCE:
      (A)LENGTH: 14 bases
      (B) TYPE: nucleid acid
      (C) NUMBER OF STRANDS: single
      (D) TOPOLOGY: linear
   (ii)- TYPE OF MOLECULE: another nucleic acid
      (A)DESCRIPTION: /desc = "synthetic DNA"
   (xi) SEQUENCE DESCRIPTION: SEQ ID NO: 1: TTTTTTTTTT TTTT
(2) INFORMATION ON SEQ ID NO.: 2:
   (i) CHARACTERISTICS OF THE SEQUENCE:
      (A) LENGTH: 21 bases
      (B) TYPE: nucleid acid
      (C) NUMBER OF STRANDS: single
      (D) TOPOLOGY: linear
   (ii) TYPE OF MOLECULE: another nucleic acid
      (A) DESCRIPTION: /desc = "synthetic DNA"
   (xi) SEQUENCE DESCRIPTION: SEQ ID NO: 2: ACCACATCAA TGTCTAGGGT GA
(2) INFORMATION ON SEQ ID NO.: 3:
   (i) CHARACTERISTICS OF THE SEQUENCE:
      (A) LENGTH: 22 bases
      (B) TYPE: nucleid acid
      (C) NUMBER OF STRANDS: single
      (D) TOPOLOGY: linear
   (ii) TYPE OF MOLECULE: another nucleic acid
      (A) DESCRIPTION: /desc = "synthetic DNA"
   (xi) SEQUENCE DESCRIPTION: SEQ ID NO: 3: CGCAATGGCA TTGGTGGGAA AC
(2) INFORMATION ON SEQ ID NO.: 4 :
   (i) CHARACTERISTICS OF THE SEQUENCE:
      (A) LENGTH: 36 bases
      (B) TYPE: nucleid acid
      (C) NUMBER OF STRANDS: single
      (D) TOPOLOGY: linear
   (ii) TYPE OF MOLECULE: another nucleic acid
      (A) DESCRIPTION: /desc.= "synthetic DNA"
   (xi) SEQUENCE DESCRIPTION: SEQ ID NO: 4: TTTTTTTTTT TTTTTTTTTT TTTTTTTTTT TTTTTT
(2) INFORMATION ON SEQ ID NO.: 5 :
   (i) CHARACTERISTICS OF THE SEQUENCE:
      (A) LENGTH: 25 bases
      (B) TYPE: nucleid acid
      (C) NUMBER OF STRANDS: single
      (D) TOPOLOGY: linear
   (ii) TYPE OF MOLECULE: another nucleic acid
      (A) DESCRIPTION: /desc = "synthetic DNA"
   (xi) SEQUENCE DESCRIPTION: SEQ ID NO: 5: GGGAACATTGG TGGAGCACGA .CACGC
(2) INFORMATION ON SEQ ID NO.: 6 :
   (i) CHARACTERISTICS OF THE SEQUENCE:
      (A) LENGTH: 34 bases
      (B) TYPE: nucleid acid
      (C) NUMBER OF STRANDS.: single
      (D) TOPOLOGY: linear
   (ii) TYPE OF MOLECULE: another nucleic acid
      (A) DESCRIPTION: /desc = "synthetic DNA"
   (xi) SEQUENCE DESCRIPTION: SEQ ID NO: 6: CCGGATCCTA GGCCTCTCCA AATGAAATGA ACTT
(2) INFORMATION ON SEQ ID NO.: 7:
   (i) CHARACTERISTICS OF THE SEQUENCE:
      (A) LENGTH: 25 bases
      (B) TYPE: nucleid acid
      (C) NUMBER OF STRANDS: single
      (D) TOPOLOGY: linear
   (ii) TYPE OF MOLECULE: another nucleic acid
      (A) DESCRIPTION: /desc = "synthetic DNA"
   (xi) SEQUENCE DESCRIPTION: SEQ ID NO: 7: TTTAAAAAAT ATAAAAACTC AACAT
(2) INFORMATION ON SEQ ID NO.: 8
   (i) CHARACTERISTICS OF THE SEQUENCE:
      (A) LENGTH: 43 bases
      (B)TYPE: nucleid acid
      (C) NUMBER OF STRANDS: single
      (D) TOPOLOGY: linear
   (ii) TYPE OF MOLECULE: another nucleic acid
      (A) DESCRIPTION: /desc = "synthetic DNA"
   (xi) SEQUENCE DESCRIPTION: SEQ ID NO: 8: GGGTAATACG ACTCACTATA GAAAAAATAT AAAAACTCAA CAT
(2) INFORMATION ON SEQ ID NO.: 9:
   (i) CHARACTERISTICS OF THE SEQUENCE:
      (A) LENGTH: 22 bases
      (B) TYPE: nucleid acid
      (C) NUMBER OF STRANDS: single
      (D)TOPOLOGY: linear
   (ii) TYPE OF MOLECULE: another nucleic acid
      (A) DESCRIPTION: /desc = "synthetic DNA"
   (xi) SEQUENCE DESCRIPTION: SEQ ID NO: 9: GACGCAAATC ACCAGTCTCT CT
(2) INFORMATION ON SEQ ID NO.: 10:
   (i) CHARACTERISTICS OF THE SEQUENCE:
      (A) LENGTH: 30 bases
      (B) TYPE: nucleid acid
      (C) NUMBER OF STRANDS: single
      (D) TOPOLOGY: linear
   (ii) TYPE OF MOLECULE: another nucleic acid
      (A) DESCRIPTION: /desc = "synthetic DNA"
   (xi) SEQUENCE DESCRIPTION: SEQ ID NO: 10: TCGAGGGCCC ACTGGATTTT GGTTTTAGGA
(2) INFORMATION ON SEQ ID NO.: 11:
   (i) CHARACTERISTICS OF THE SEQUENCE:
      (A) LENGTH: 28 bases
      (B) TYPE: nucleid acid
      (C) NUMBER OF STRANDS: single
      (D) TOPOLOGY: linear
   (ii) TYPE OF MOLECULE: another nucleic acid
      (A) DESCRIPTION: /desc = "synthetic DNA"
   (xi) SEQUENCE DESCRIPTION: SEQ ID NO: 11: GACGATCTAG TAACATAGAT GACACCGC,
(2) INFORMATION ON SEQ ID NO.: 12 :
   (i) CHARACTERISTICS OF THE SEQUENCE:
      (A) LENGTH: 29 bases
      (B) TYPE: nucleid acid
      (C) NUMBER OF STRANDS: single
      (D) TOPOLOGY: linear
   (ii) TYPE OF MOLECULE: another nucleic acid
      (A) DESCRIPTION: /desc = "synthetic DNA"
   (xi) SEQUENCE DESCRIPTION: SEQ ID NO: 12: TCGAGGGCCC GATCGTTCAA ACATTTGGC

## Claims

1. An infectious clone of the UK1 isolate of the turnip mosaic virus (TuMV), which comprises:
- a complete copy of the complementary DNA (cDNA) to the genomic RNA of TuMV UK1, in the form of double stranded DNA as contained in the plasmids deposited on 19.12.1996 at the Colección Española de Cultivos Tipo (CECT) with numbers CECT 4822 or CECT 4823.
- a transcription promoter sequence operably linked to said cDNA, said promoter sequence being selected from the group consisting of a cauliflower mosaic virus (CaMV).35S promoter and a T7 RNA polymerase promoter; and
- a replicon.

2. An infectious clone according to claim 1, in which said transcription promoter sequence comprises the promoter sequence of bacteriophage T7 and a additional G adjacent to the first nucleotide at the 5'end of the cDNA-if said cDNA does not begin by G.

3. An infectious clone according to claim 1, which also comprises a transcription termination sequence located at the 3'end of the poly(A) tail.

4. An infectious clone according to claim 3, in which said transcription termination sequence comprises the terminator sequence of the Ti plasmid nos gene of the Agrobacterium tumefaciens.

5. An infectious clone according to claim 1, which also comprises a polyadenilation signal sequence at the 3'end.

6. An infectious clone according to claim 5, in which said polyadenilation signal sequence comprises the polyadenilation signal sequence of the 35S gene from the cauliflower mosaic virus (CaMV).

7. An infectious clone according to any claims 3 through 6, which also compises a variable number of nucleotides between the last nucleotide at the 3'end of the poly(A) tail and the first nucleotide of the transcription termination sequence of the polyadenilation signal.

8. An infectious clone according to claim 1, in which said replicon is a plasmid capable of replicating inside of an adequate host cell.

9. An infectious clone according to claim 8, in which said adequate host cell is a bacteria.

10. An infectious clone according to claim 9, in which said bacteria is Escherichia coli.

11. An infectious clone according to claim 1, which comprises the full length cDNA corresponding to the genome of TuMV UK1 under the control of the promoter of the T7 bacteriophage.

12. An infectious clone according to claims 1, which comprises the full length cDNA corresponding to the genome of TuMV UK1 under the control of the 35S promoter of CaMV.

13. An infectious clone according to claim 12, which also comprises the 35S polyadenilation signal of CaMV.

14. An infectious clone according to claim 12, which also comprises the terminator of the nos gene.

15. A procedure for the obtaining of an infectious clone of the turnip mosaic virus (TuMV) UK1 according to claims 1 through 14 which comprises the construction of the full length complementary DNA (cDNA) to the genomic RNA of TuMV UK1 and the assemblage of the transcription promoter elements.

16. A recombinant viral vector derived from a turnip mosaic virus (TuMV) UK1 infectious clone which comprises a TuMV UK1 infectious clone according to claims 1 through 14 and an heterologous DNA sequence.

17. A method for infecting plants which comprises putting n contact an infectious clone of the turnip mosaic virus (TuMV) UK1 according to claims 1 through 14 or a viral infectious vector according to claim 16 with a plant susceptible of being infected by TuMV.

18. A method according to claim 17, in which said plant belongs to the Arabidopsis thaliana. (L) Heynh species.

## Patentansprüche

1. Ein infektiöser Klon des UK1-Isolates des Turnip Mosaic Virus (TuMV), welcher Folgendes umfasst:
- eine vollständige Kopie einer zu der genomischen RNA des TuMV UK1 komplementären DNA (cDNA), in der Form einer doppelsträngigen DNA, wie sie in den Plasmiden enthalten sind, die am 19.12.1996 bei der spanischen Hinterlegungsstelle Colección Española de Cultivos Tipo (CECT) mit den Nummern CECT 4822 oder CECT 4823 hinterlegt wurden.
- eine Transkriptionspromotorsequenz, die funktionsfähig mit der besagten cDNA verbunden ist, wobei die besagte Promotorsequenz aus der Gruppe ausgewählt ist, die aus einem Cauliflower Mosaic Virus (CaMV), einem 35S-Promotor und einem T/-RNA-Polymerase-Promotor besteht, und
- ein Replikon.

2. Ein infektiöser Klon nach Anspruch 1, bei dem die besagte Transkriptionspromotorsequenz die Promotorsequenz Bakteriophage T7 und ein ergänzendes G, das an das erste Nukleotid am 5'-Ende der cDNA angrenzt, wenn die besagte cDNA nicht bei G beginnt, umfasst.

3. Ein infektiöser Klon nach Anspruch 1, der ebenso eine Transkriptionsterminierungssequenz, die sich am 3'-Ende des Poly(A)-Endes befindet, umfasst.

4. Ein infektiöser Klon nach Anspruch 3, bei dem die besagte Transkriptionsterminierungssequenz die Terminatorsequenz des Ti-Plasmids-nos-Gens des *Agrobacterium tumefaciens* umfasst.

5. Ein infektiöser Klon nach Anspruch 1, der ebenso eine Polyadenylationsignalsequenz am 3'-Ende umfasst.

6. Ein infektiöser Klon nach Anspruch 5, bei dem die besagte Polyadenylationsignalsequenz die Polyadenylationsignalsequenz des 35S-Gens von dem Cauliflower Mosaic Virus (CaMV) umfasst.

7. Ein infektiöser Klon nach einem der Ansprüche 3 bis einschließlich 6, der ebenso eine variable Anzahl von Nukleotiden zwischen dem letzten Nukleotid an dem 3'-Ende des Poly(A)-Endes und dem ersten Nukleotid der Transkriptionsterminierungssequenz des Polyadenylationsignals umfasst.

8. Ein infektiöser Klon nach Anspruch 1, bei dem das besagte Replikon ein Plasmid ist, das in der Lage ist, sich in einer geeigneten Wirtszelle zu replizieren.

9. Ein infektiöser Klon nach Anspruch 8, bei dem die besagte geeignete Wirtszelle eine Bakterie ist.

10. Ein infektiöser Klon nach Anspruch 9, bei dem besagte Bakterie *Escherichia coli* ist.

11. Ein infektiöser Klon nach Anspruch 1, der die gesamte Länge cDNA, die dem Genom des TuMV UK1 unter der Kontrolle des Promotors der T7 Bakteriophage entspricht, umfasst.

12. Ein infektiöser Klon nach Anspruch 1, der die gesamte Länge cDNA, die dem Genom des TuMV UK1 unter der Kontrolle des 3SS-Promotors des CaMV entspricht, umfasst

13. Ein infektiöser Klon nach Anspruch 12, der ebenso das 3SS-Polyadenylationsignal des CaMV umfasst.

14. Ein infektiöser Klon nach Anspruch 12, der ebenso den Terminator des nos-Gens umfasst.

15. Ein Verfahren zur Gewinnung eines infektiösen Klons des Turnip Mosaic Virus (TuMV) UK1 nach Anspruch 1 bis einschließlich 14, das die Konstruktion der zu der genomischen RNA des TuMV UK1 komplementären DNA (cDNA) in seiner gesamten Länge und die Assemblage der Transkriptionspromotorelemente umfasst.

16. Ein rekombinanter viraler Vektor, der von einem Turnip Mosaic Virus (TuMV) UK1 infektiösen Klon abgeleitet ist, welcher einen TuMV UK1 infektiösen Klon gemäß den Ansprüchen 1 bis einschließlich 14 und eine heterologe DNA-Sequenz umfasst.

17. Ein Verfahren zur Infizierung von Pflanzen, welches die Inkontaktsetzung eines infektiösen Klons des Turnip Mosaic Virus (TuMV) UK1 gemäß Anspruch 1 bis einschließlich 14 oder eines viralen infektiösen Vektors gemäß Anspruch 16 mit einer Pflanze, die durch den TuMV infiziert werden kann, umfasst.

18. Ein Verfahren nach Anspruch 17, bei dem die besagte Pflanze zu der Art der *Arabidopsis thaliana* (L) Heynh gehört.

## Revendications

1. Un clone infectieux de l'isolat UK1 du virus de la mosaïque du navet (TuMV), qui comprend :
- une copie complète de l'ADN complémentaire (ADNc) à l'ARN génomique du TuMV UK1, sous la forme d'ADN double brin tel qu'il est contenu dans les plasmides déposés le 19.12.1996 à la Collection Espagnole de Cultures Type (CECT) sous les numéros CECT 4822 ou CECT 4823.
- une séquence promotrice de transcription fonctionnellement liée audit ADNc, ladite séquence promotrice étant choisie parmi le groupe constitué d'un promoteur 35S du virus de la mosaïque du chou-fleur (CaMV) et d'un promoteur de polymérase d'ARN T7 ; et
- un réplicon.

2. Un clone infectieux selon la revendication 1, dans lequel ladite séquence promotrice de transcription comprend la séquence promotrice de bactériophage T7 et un G supplémentaire adjacent au premier nucléotide à l'extrémité 5' de l'ADNc si ledit ADNc ne commence pas par G.

3. Un clone infectieux selon la revendication 1, qui comprend aussi une séquence de terminaison de transcription située à l'extrémité 3' de la queue poly(A).

4. Un clone infectieux selon la revendication 3, dans lequel ladite séquence de terminaison de transcription comprend la séquence de terminateur du gène nos du plasmide Ti de l'Agrobacterium tumefaciens.

5. Un clone infectieux selon la revendication 1, qui comprend aussi une séquence de signal de polyadénylation à l'extrémité 3'.

6. Un clone infectieux selon la revendication 5, dans lequel ladite séquence de signal de polyadénylation comprend la séquence de signal de polyadénylation du gène 35S du virus de la mosaïque du chou-fleur (CaMV).

7. Un clone infectieux selon quelconque des revendications 3 à 6 qui comprend aussi un nombre variable de nucléotides entre le dernier nucléotide à l'extrémité 3' de la queue poly(A) et le premier nucléotide de la séquence de terminaison de transcription du signal de polyadénylation.

8. Un clone infectieux selon la revendication 1, dans lequel ledit réplicon est un plasmide capable de réplication à l'intérieur d'une cellule hôte appropriée.

9. Un clone infectieux selon la revendication 8, dans lequel ladite cellule hôte appropriée est une bactérie.

10. Un clone infectieux selon la revendication 9, dans lequel ladite bactérie est l'Escherichia coli.

11. Un clone infectieux selon la revendication 1, qui comprend l'ADNc pleine longueur correspondant au génome du TuMV UK1 sous le contrôle du promoteur du bactériophage T7.

12. Un clone infectieux selon la revendication 1, qui comprend l'ADNc pleine longueur correspondant au génome du TuMV UK1 sous le contrôle du promoteur 35S du CaMV.

13. Un clone infectieux selon la revendication 12, qui comprend aussi le signal de polyadénylation 35S du CaMV.

14. Un clone infectieux selon la revendication 12, qui comprend aussi le terminateur du gène nos.

15. Une procédure pour l'obtention d'un clone infectieux du virus de la mosaïque du navet (TuMV) UK1 selon les revendications 1 à 14 qui comprend la construction de l'ADN complémentaire (ADNc) pleine longueur à l'ARN génomique du TuMV UK1 et l'assemblage des éléments promoteurs de transcription.

16. Un vecteur viral recombiné dérivé d'un clone infectieux du virus de la mosaïque du navet (TuMV) UK1 qui comprend un clone infectieux (TuMV) UK1 selon les revendications 1 à 14 et une séquence ADN hétérologue.

17. Un procédé pour infecter les plantes qui comprend le fait de mettre en contact un clone infectieux du virus de la mosaïque du navet (TuMV) UK1 selon les revendications 1 à 14 ou un vecteur infectieux viral selon la revendication 16 avec une plante susceptible d'être infectée par le TuMV.

18. Un procédé selon la revendication 17, dans lequel ladite plante appartient aux espèces d'Arabidopsis thaliana (L.) Heynh.
